# EUROPEAN PATENT APPLICATION

(11) **EP 4 316 515 A1**
(43) Date of publication of application: **07.02.2024**
(21) Application number: 22780974.6
(22) Date of filing: 29.03.2022
(51) Int. Cl.: A61K 39/145, A61K 39/00, A61P 31/16

(54) **INFLUENZA VACCINE**

(30) Priority: 30.03.2021 JP 2021057975
(71) Applicant: Denka Company Limited, Tokyo 103-8338 (JP)
(72) Inventor: MITSUMATA, Ryotaro, Gosen-shi, Niigata 959-1695 (JP); NARAHARA, Tomohiro, Gosen-shi, Niigata 959-1695 (JP)
(74) Representative: Wächtershäuser & Hartz Patentanwaltspartnerschaft mbB
(86) International application number: PCT/JP2022/015638
(87) International publication number: WO 2022/210763

(57) **Abstract**

Provided is an influenza vaccine that has high immunogenicity in children and elderly people and has a higher efficacy than conventional split vaccines. An influenza vaccine composition to be administered to children and/or elderly people, comprising, as a virus antigen, inactivated whole particles subjected to inactivation treatment with beta-propiolactone.

## Description

### Technical Field

The present invention relates to an influenza vaccine having a high efficacy for children or elderly people.

### Background Art

Human influenza virus is a single-stranded RNA virus of the family Orthomyxoviridae, and is classified into types A, B, and C depending on antigenicity of internal antigens. Among them, type A and type B cause large epidemics every year mainly in winter, and it is known that about 10 million or more people are infected per year in Japan. The most effective means for preventing the influenza virus that causes such large epidemics every year is a vaccine. The influenza vaccine in Japan was introduced in response to Asian influenza that prevailed in 1957, and at that time, it was an inactivated whole-virus vaccine obtained by inactivation treatment on the influenza virus purified. However, thereafter, in order to reduce side reactions such as a fever, split vaccines obtained by disrupting virus particles by treatment with diethyl ether or a surfactant and removing lipid components have been approved, and the split vaccines have been now widely used worldwide.

However, the split vaccines are known to have low immunogenicity particularly in age groups with weak immune response such as children and elderly people. Therefore, about half of infections of a seasonal influenza virus are in children; although a frequency is low, an onset risk of influenza encephalopathy is higher in children than in other age groups; and infection symptoms tend to be severe and a risk of death is high in elderly people. Therefore, in Europe and the United States, the influenza vaccines having a high efficacy for children and elderly people have been approved, such as live attenuated nasal vaccines, adjuvant preparations, and high-titer vaccines.

One of reasons why immune response is weak in elderly people is a decrease in innate immunity in elderly people. In particular, Toll-like receptors (TLRs) in phagocytic cells play an important role in connecting the innate immunity and acquired immunity (Non Patent Literature 1), but it has been found that expression of the TLRs is significantly lowered in macrophages of elderly people, and thus the acquired immune response as an antigen-specific reaction is also decreased (Non Patent Literature 2). Furthermore, it is necessary to promote a stronger primary immunity in children with no previous history of infection and poor vaccination history, and thus strong activation of plasmacytoid dendritic cells via TLR7 is required (Non Patent Literature 3).

### Citation List

### Non Patent Literature

Non Patent Literature 1: Vasselon T, Detmers PA. Toll receptors: a central element in innate immune responses. Infect Immun. 2002 Mar;70(3):1033-41.
Non Patent Literature 2: Renshaw M, Rockwell J, Engleman C, Gewirtz A, Katz J, Sambhara S. Cutting edge: impaired Toll-like receptor expression and function in aging. J Immunol. 2002 Nov 1;169(9):4697-701.
Non Patent Literature 3: Akira S. Innate immunity and adjuvants. Philos Trans R Soc Lond B Biol Sci. 2011 Oct 12;366 (1579) :2748-55.

### Summary of Invention

### Technical Problem

The present invention relates to provision of an influenza vaccine that has high immunogenicity in children and elderly people and has a higher efficacy than conventional split vaccines.

### Solution to Problem

As a result of intensive studies, the inventors of the present application have found that by using beta-propiolactone for the inactivation treatment of the influenza virus, it is possible to prepare an inactivated whole-virus vaccine that maintains high innate immune activity as compared to a conventional treatment with formalin, and the vaccine has higher immunogenicity than the split vaccine approved in pediatric and elderly model animals. Then, the inactivated whole-virus vaccine treated with the beta-propiolactone can induce a virus-specific antibody to nasal mucosa in which the virus proliferates, and a significantly high antibody induction has been confirmed over the split vaccine in the pediatric and elderly model animals.

Accordingly, the present invention relates to 1) to 6) below.
1) An influenza vaccine composition to be administered to children and/or elderly people, containing, as a virus antigen, inactivated whole particles subjected to inactivation treatment with beta-propiolactone.
2) The composition according to 1), in which the virus antigen contains a type-A influenza virus strain including an A/H1N1 subtype and an A/H3N2 subtype, and a type-B influenza virus strain including a B/Victoria lineage and a B/Yamagata lineage.
3) The composition according to 1) or 2), containing no adjuvant.
4) The composition according to any one of 1) to 3), in which the composition is subcutaneously administered.
5) An immunization method including administering a composition containing, as a virus antigen, inactivated whole particles subjected to inactivation treatment with beta-propiolactone to children or elderly people.
6) An influenza prevention method including administering a composition containing, as a virus antigen, inactivated whole particles subjected to inactivation treatment with beta-propiolactone to children or elderly people.

### Advantageous Effects of Invention

According to the present invention, provided is an influenza vaccine showing a higher efficacy for children and/or elderly people with weak immune response than the approved split vaccine. Since the influenza vaccine composition of the present invention promotes a high antibody induction in both serum and nasal mucosa in children and elderly people, it is possible to enhance protective activity against virus infection in children and elderly people with high risk of infection and exacerbation, and it is possible to greatly contribute to pharmaceutical industry in creation of an influenza preventive drug with a high value added.

### Brief Description of Drawings

Fig. 1 shows an influence on innate immune activity of an influenza vaccine by inactivation treatment. BPL: beta-propiolactone.
Fig. 2A shows an IgG titer against an A/Singapore/GP1908/2015 strain in serum after 21 days from subcutaneous administration of a vaccine.
Fig. 2B is an IgG titer against a B/Phuket/3073/2013 strain in serum after 21 days from subcutaneous administration of a vaccine.
Fig. 3A shows an IgG titer against an A/Singapore/GP1908/2015 strain in serum after 42 days from subcutaneous administration of a vaccine.
Fig. 3B shows an IgG titer against a B/Phuket/3073/2013 strain in serum after 42 days from subcutaneous administration of a vaccine.
Fig. 4A shows a neutralizing antibody titer against an A/Singapore/GP1908/2015 strain in serum after 42 days from subcutaneous administration of a vaccine.
Fig. 4B shows a neutralizing antibody titer against a B/Phuket/3073/2013 strain in serum after 42 days from subcutaneous administration of a vaccine.
Fig. 5A shows an IgG titer against an A/Singapore/GP1908/2015 strain in nasal wash after 42 days from subcutaneous administration of a vaccine.
Fig. 5B shows an IgG titer against a B/Phuket/3073/2013 strain in nasal wash after 42 days from subcutaneous administration of a vaccine.

### Description of Embodiments

Hereinafter, preferred embodiments of the present invention will be described in detail. However, the present invention is not limited to the embodiments below.

The virus antigen in the influenza vaccine composition of the present invention refers to whole influenza virus particles subjected to the inactivation treatment with the beta (β) propiolactone.

In the present invention, the term "influenza virus" refers to influenza A virus or influenza B virus, or both of them. Furthermore, the influenza virus also includes all currently known subtypes and subtypes to be isolated and identified in future.

The virus antigen of the present invention includes at least either influenza A virus or influenza B virus. In other words, the influenza vaccine of the present invention may be a monovalent vaccine containing the virus antigen of either one of influenza A virus and influenza B virus, or may be a multivalent vaccine containing both of the virus antigens.

Examples of the virus antigen include virus antigens containing an influenza A virus strain including the A/H1N1 subtype and/or the A/H3N2 subtype and an influenza B virus strain including the B/Victoria lineage and/or the B/Yamagata lineage, and more preferably, virus antigens containing two strains of the type-A (the A/H1N1 subtype and the A/H3N2 subtype) and two strains of the type-B (the B/Victoria lineage and the B/Yamagata lineage).

The whole influenza virus particles can be prepared by a method using an embryonated chicken egg or cultured cell lines.

The method for preparation with the embryonated chicken egg is, for example, a method including inoculating an embryonated chicken egg with a virus strain and culturing the resultant, and thereafter clarifying, concentrating, and purifying the virus suspension to thereby obtain a virus solution containing virus particles. Furthermore, the method for preparation with the cultured cells is, for example, a method including inoculating cultured cell lines with a virus strain and culturing the resultant, and thereafter obtaining a virus solution containing virus particles from the culture supernatant in a similar manner as in the method with the embryonated chicken egg described above.

Here, the culture is carried out by inoculating the cell lines with the influenza virus strain and culturing at 30 to 37°C for about 1 to 7 days, preferably at 33 to 35°C for about 2 days. After completion of the culture, the virus suspension (an infected allantoic fluid or an infected cell culture supernatant) is collected and centrifuged or filtered for clarification. Next, for concentration, a barium salt adsorption elution reaction or ultrafiltration is performed. The purification of the virus can be performed by using a means including ultracentrifugation such as sucrose density gradient centrifugation, or liquid chromatography.

The cultured cells are not particularly limited as long as the influenza virus exhibits proliferation property, and examples thereof include MDCK (Madin-Darby Canine Kidney), Vero, Caco-2, PER.C6, EB66, and recombinant cells thereof that have been recombined so as to highly express receptors used for entry by the virus.

The whole influenza virus particles thus obtained are subjected to the inactivation treatment by beta-propiolactone.

In other words, in the present invention, the inactivation treatment with beta-propiolactone is performed instead of a conventional inactivation treatment using formaldehyde (formalin treatment).

Beta-propiolactone is a widely used monoalkylating agent for virus inactivation in preparation of many vaccines.

The inactivation treatment with beta-propiolactone is performed at any time of culturing with the embryonated chicken egg or the cultured cells, and clarifying, concentrating or purifying the influenza virus suspension collected.

Examples of the treatment with beta-propiolactone include a method for adding β-propiolactone to the virus solution collected after the culture so as to have a final concentration of 0.0125 to 0.1 vol%, preferably 0.025 to 0.075 vol%, more preferably 0.05 vol% and carrying out a reaction at 2 to 8°C for 18 hours or more, preferably 20 hours or more, more preferably 24 hours or more, and 50 hours or less, preferably 30 hours or less.

As a more preferred aspect, for example, treatment at 2 to 8°C and 0.05% for 24 hours can be exemplified.

As shown in examples to be described later, the influenza vaccine containing, as an antigen, inactivated whole virus particles subjected to the inactivation treatment with beta-propiolactone has a higher innate immunity activation ability than the inactivated whole influenza vaccine prepared by the conventional formalin treatment, and shows a significantly higher antibody induction than an existing split vaccine in serum after a first and second administration, by administering the influenza vaccine to the pediatric and elderly model animals. Furthermore, after the second administration, the vaccine shows a high virus-specific IgG induction also in the nasal mucosa, and shows a significantly higher antibody induction than the split vaccine for both the type-A virus and the type-B virus in the pediatric model animals, and a significantly higher antibody induction than the existing vaccine for the type-B virus in the elderly model animals.

Accordingly, the composition containing, as the virus antigen, the whole influenza virus particles subjected to the inactivation treatment with beta-propiolactone is useful as an influenza vaccine exhibiting a high innate immunity activation ability in a case where the composition is administered to children and/or elderly people. Furthermore, administration of the composition containing, as the virus antigen, the whole influenza virus particles subjected to the inactivation treatment with beta-propiolactone to children and/or elderly people is useful as an immunization method for inducing antibodies against the influenza virus in children and/or elderly people, and as the influenza prevention method.

Note that, the innate immunity activation ability can be evaluated by TLR activation ability, and for example, as described in examples below, the evaluation can be performed by measuring SEAP activity (RLU value: Relative Light Unit) in the culture supernatant when the influenza antigen is exposed to RAW 264.7 cells into which TLR7 genes and secreted alkaline phosphatase (SEAP) genes are incorporated.

The virus antigen of the influenza vaccine composition of the present invention preferably has, for example, an RLU value measured by using recombinant RAW 264.7 cells of 50 or more, and more preferably 60 or more, as shown in examples to be described later.

The influenza vaccine composition of the present invention can contain, in addition to the influenza antigen described above, a pharmaceutically acceptable carrier as appropriate, and can be formulated into a predetermined form.

Here, examples of the carrier include a carrier usually used for producing a vaccine, and specific examples thereof include a buffer, an emulsifier, a preservative (for example, thimerosal), an isotonizing agent, a pH adjusting agent, a thickening agent, an inactivating agent (for example, the formalin), an adjuvant, and an immunostimulant. The adjuvant refers to a substance that enhances the immune response to an antigen by administering the adjuvant together with the antigen, but since the vaccine antigen itself has high antibody inducibility as described above, the adjuvant is not necessarily added and the vaccine composition of the present invention may be an adjuvant-free composition.

Such influenza vaccine composition is suitably formulated in a solid, semi-solid or liquid form to suit an administration route intended. Examples of the administration route as appropriate include intramuscular, transdermal, subcutaneous, intradermal, intranasal, sublingual, and oral, and subcutaneous administration is preferable. Examples of a dosage form administrated by injection such as intramuscular, transdermal, subcutaneous, or intradermal injection include a liquid agent, an emulsion, a water-soluble or hydrophobic suspension, and dry powder used by adding a liquid and dissolving or suspending the dry powder.

A content of the influenza antigen in the influenza vaccine composition described above is 7.5 µg or more per virus strain, that is, 7.5 µg HA/strain or more, preferably 9 to 21 µg HA/strain, and more preferably 15 µg HA/strain as an amount of hemagglutinin per administration unit. Note that, the amount of hemagglutinin is a value obtained by measurement by a test method defined in accordance with WHO or national standards, such as a single radial immunodiffusion test method. Furthermore, a content of the antigen contained in the vaccine may be appropriately varied according to the type of the virus or subjects to be administrated.

The subjects to be administered with the influenza vaccine composition of the present invention are children and/or elderly people. In the present invention, the term "children" means a human of 0 years old or more and less than 15 years old, preferably 3 years old or more and less than 12 years old, and the term "elderly people" means a human of preferably 50 years old or more, more preferably 55 years old or more, and still more preferably 60 years old or more.

The number of times for administering the influenza vaccine composition of the present invention is at least once, but is preferably at least twice from a viewpoint of the efficacy. Further administration is sometimes referred to as booster, and thus a more effective protective effect of infection can be achieved. An interval of the booster is recommended to be at least 1 week, and an interval of 1 to 4 weeks is preferable.

### Examples

Hereinafter, the present invention will be specifically described with reference to examples, but the present invention is not limited thereto at all.

### Example 1 Evaluation of influence on innate immunity activation ability by inactivation treatment

A virus of the B/Phuket/3073/2013 strain was inoculated into a chorioallantoic cavity of a 12-day-old embryonated chicken egg and cultured for 3 days, and the chorioallantoic fluid was sampled. The chorioallantoic fluid sampled was clarified by filter filtration, then adsorbed to barium sulfate, and eluted with a 12% sodium citrate solution to collect the influenza virus. The virus collected was further purified by replacing the solution with 6.7 mM phosphate buffered saline (pH 7.2) by ultrafiltration, and collecting a fraction containing the influenza virus by sucrose density gradient centrifugation after buffer replacement. The virus solution obtained was used as a purified influenza virus solution.

Beta-propiolactone (BPL) as an inactivating agent was added to the purified influenza virus solution to have a final concentration of 0.05%, and infectivity of the influenza virus was inactivated by a reaction at 4°C for 24 hours, after the reaction for the inactivation, a buffer was replaced with 6.7 mM phosphate buffered saline (pH 7.2) by ultrafiltration (MWCO: 100,000), and regarded as a β-propiolactone-treated inactivated whole-virus vaccine.

Furthermore, the purified influenza virus solution was adjusted to have a protein concentration of 200 ug/mL, a 10% neutral buffered formalin solution (FUJIFILM Wako Pure Chemical Corporation, a content of the formaldehyde: 3.8 to 4.1%) was added to allow the formalin to have a final concentration of 0.01 or 0.02%, and a reaction was performed at 4°C for 14 days. After the reaction, glycine was added to have a final concentration of 10 mM to stop the reaction of formaldehyde, and the influenza virus solution was centrifuged at 4°C and 1,000,000 × g for 4 hours. A supernatant was discarded after the centrifugation, and a pellet of the virus obtained was suspended in 6.7 mM phosphate buffered saline (pH 7.2), and regarded as a formaldehyde-treated inactivated whole-virus vaccine.

To 1.5 × 10⁶ cells of NBP2-26261 (Novus biologicals: a recombinant RAW 264.7 cell obtained by incorporating a secreted alkaline phosphatase (SEAP) gene into the downstream of a transcription response sequence of NFκB, and expressing all other TLRs except TLR5), 10 µg as a total amount of protein of various inactivated vaccines were added, and cultured for 24 hours under conditions of 37°C and 5% CO₂. After the culture, the supernatant was collected, and alkaline phosphatase activity of the supernatant was measured with a SEAP Reporter Assay Kit (a trade name, Cayman). Since the value (RLU: Relative Light Unit) measured is an index of the innate immune activity via the TLR, the innate immune activation ability of the vaccine prepared under each inactivation treatment condition was compared with the value measured.

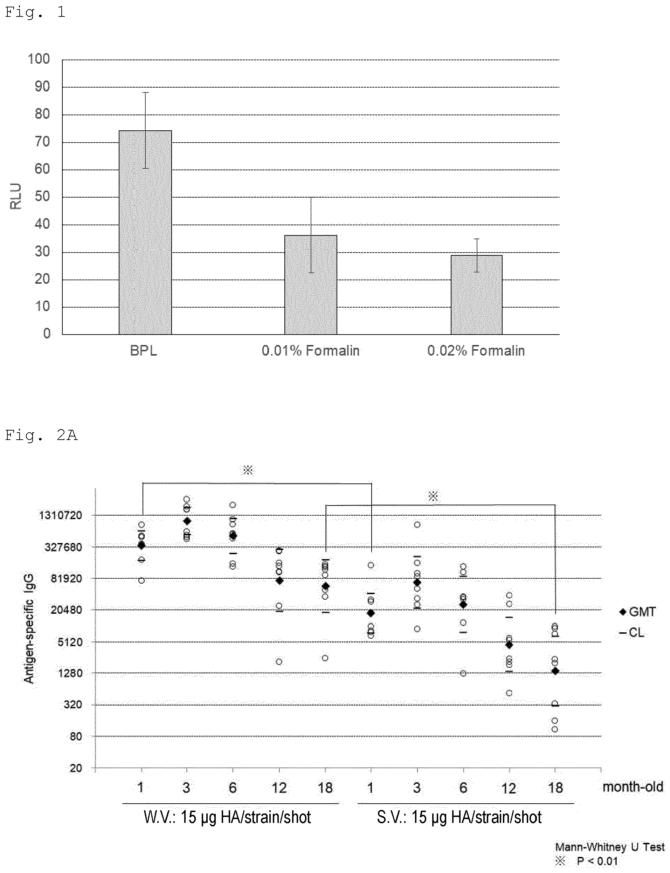

The comparison of the values measured is as illustrated in Fig. 1, and in a reaction of the 0.02% formalin at 4°C for 14 days as the general inactivation treatment conditions of the virus, a clear decrease in innate immunity activation ability was confirmed. Furthermore, also in the treatment with 0.01% formalin, the innate immunity activation ability was showed lower than the treatment with beta-propiolactone although the value was higher than that of the treatment with 0.02% formalin. Accordingly, it was shown that the inactivated whole-virus vaccine treated with beta-propiolactone has a high innate immunity activation ability as compared to the inactivated whole-virus vaccine obtained by inactivated with formalin that was distributed before 1971.

### Example 2 Evaluation of immunogenicity in pediatric and elderly mouse models

Similarly to Example 1, a β-propiolactone-treated inactivated whole-virus vaccine for the A/H1N1 subtype (the A/Singapore/GP1908/2015 strain) and the B/Yamagata lineage (the B/Phuket/3073/2013 strain) was prepared, adjusted with 6.7 mM phosphate buffered saline (pH 7.2) containing 1 w/w% sucrose to allow the hemagglutinin of each strain to be 15 µg HA per 0.2 mL, and regarded as the inactivated whole-virus vaccine. Furthermore, the split vaccine was adjusted with 6.7 mM phosphate buffered saline (pH 7.2) containing the 1 w/w% sucrose to allow the hemagglutinin of each strain to be 15 µg HA per 0.2 mL similarly, by using a stock solution of an influenza HA vaccine.

An administration solution (Table 1) prepared as described above was subcutaneously administered to 1, 3, 6, 12 and 18-month-old male C57BL/6 mice (Charles River Laboratories, Inc.) twice at intervals of 3 weeks per mouse. After 21 days (Day 21) from the first administration, partial blood was collected from tail veins, and after 21 days (Day 42) from the second administration, whole blood was collected under anesthesia. The blood collected was centrifuged to prepare serum, and subjected to various antibody titer measurement.

Furthermore, after the whole blood collection on Day 42, a Pipetman was inserted from a pharynx side of an upper jaw, D-PBS containing 0.175% BSA and a protease inhibitor (Thermo Fisher Scientific, Inc.) was poured thereinto, and a solution collected from external nostrils was used as the nasal wash. The nasal wash was also subjected to virus-specific IgG titer measurement.

**[Table 1]**

| Antigen | Vaccine strain | Administration amount (µg HA/strain/head) | Administration volume (mL) | Administration route | N |
|---|---|---|---|---|---|
| inactivated whole-virus vaccine (W.V.) | A/Singapore/GP1908/2015 B/Phuket/3073/2013 | 15 | 0.2 | Subcutaneous | 8 |
| Split vaccine (S.V.) | A/Singapore/GP1908/2015 B/Phuket/3073/2013 | 15 | 0.2 | Subcutaneous | 8 |

It is known that among the 1, 3, 6, 12, and 18-month-old C57BL/6 mice, the 1-month-old mice are immature individuals (equivalent to 12-year-old humans), the 3 and 6-month-old mice are mature individuals (equivalent to 20 to 30-year-old humans), the 12 month-old mice are middle-aged individuals (equivalent to 42-year-old humans), and the 18-month-old mice are elderly individuals (equivalent to 56-year-old humans) (technology Information from Charles River Laboratories, Inc., elderly C57BL/6J mice used in research, https://www.crj.co.jp/cms/crj/pdf/product/rm/brochure/White _Paper_B6-Aged.pdf). Accordingly, the 1-month-old mice correspond to the pediatric model animals, and the 18-month-old mice correspond to the elderly model animals.

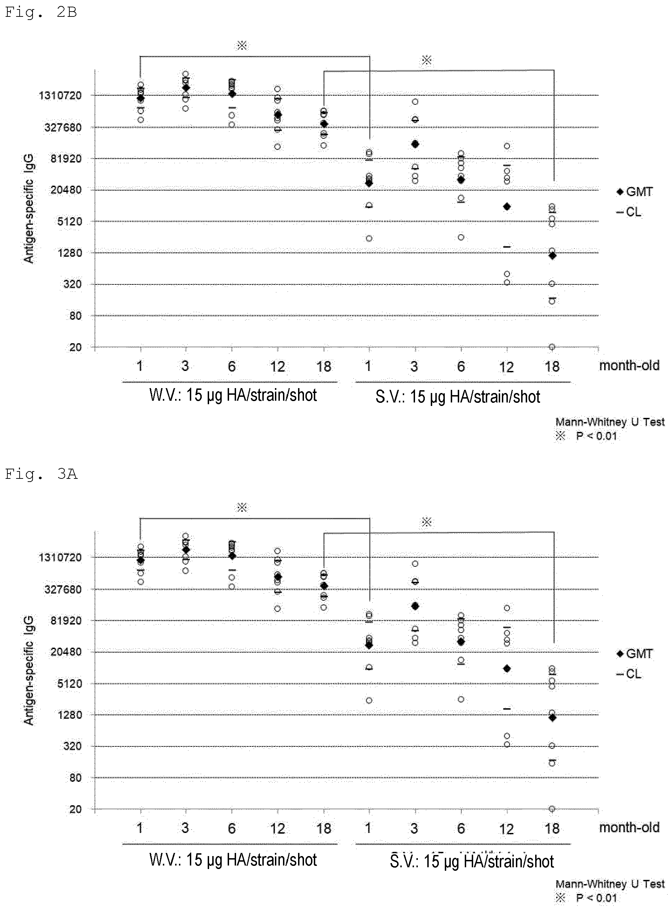

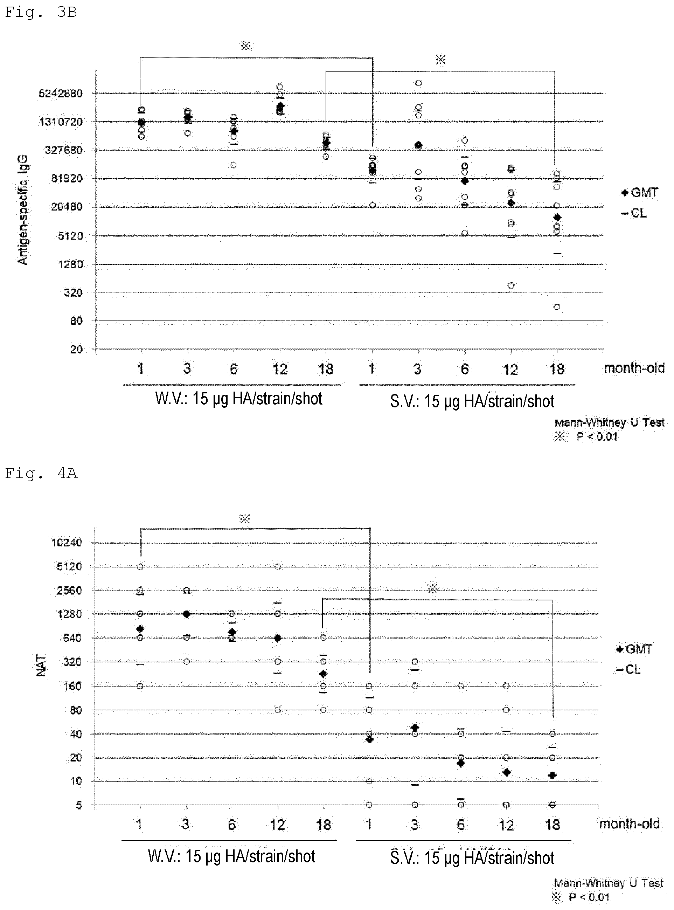

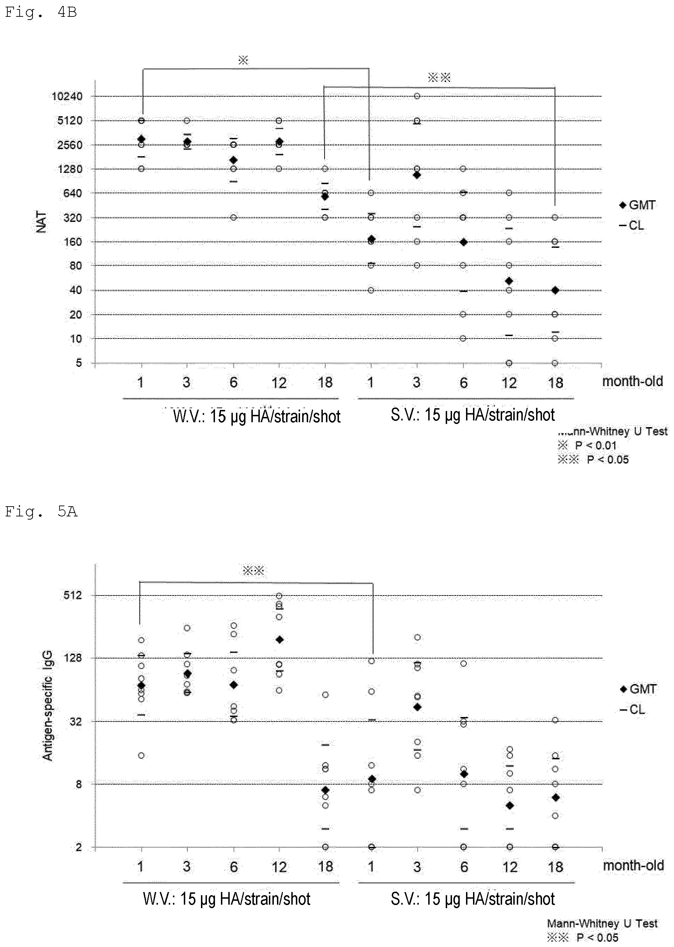

In order to confirm that the settings of these model animals are also appropriate for the immunogenicity of the influenza vaccine, the antibody induction of the split vaccine (S.V.) approved was compared in the 1 to 18-month-old mice. As a result, as illustrated in Fig. 2A (the type-A virus) and Fig. 2B (the type-B virus), the virus-specific IgG titers of the serums after the first administration were the highest in the 3-month-old mice as the mature individuals against all the strains, and the antibody induction was lower in the 1-month-old mice and the 18-month-old mice than in the 3-month-old mice. Furthermore, it was found that the results of the virus-specific IgG titers (Fig. 3A (the type-A virus) and Fig. 3B (the type-B virus)) and the neutralizing antibody titers (Fig. 4A (the type-A virus) and Fig. 4B (the type-B virus)) after the second administration were all similar to the results of the virus-specific IgG titers after the first administration, and the antibody induction was lower in the 1 and 18-month-old mice than in the 3-month-old mice as a peak of the antibody titer. Accordingly, a decrease in antibody induction was confirmed in the 1-month-old mice as the pediatric models and the 18-month-old mice as the elderly models similarly to the humans, and these models were considered to be appropriate also for the immunogenicity of the influenza vaccine.

As results of an immunogenicity test in these models, it was found from the virus-specific IgG titers of the serums after the first administration (Fig. 2A (the type-A virus) and Fig. 2B (the type-B virus)), that the antibody induction was significantly higher in an inactivated whole-virus vaccine-administered group (W.V.) than in a split vaccine-administered group (S.V.) among the 1-month-old mice and the 18-month-old mice against all the virus strains. Furthermore, the virus-specific IgG titers of the serums after the second administration also showed significantly high in the inactivated whole-virus vaccine-administered group (W.V.) similarly to the results after the first administration (Fig. 3A (the type-A virus) and Fig. 3B (the type-B virus) . For the serums after the second administration, the neutralizing antibody titers were also measured, and as illustrated in Fig. 4A (the type-A virus) and Fig. 4B (the type-B virus), the inactivated whole-virus vaccine-administered group (W.V.) showed a significantly high antibody induction among the 1-month-old mice and the 18-month-old mice.

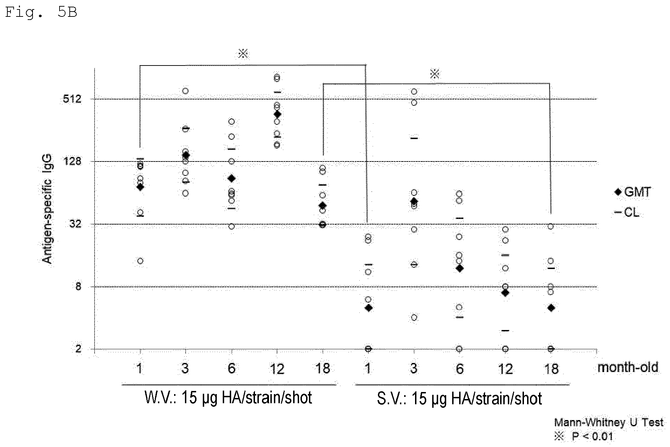

Furthermore, on Day 42 after the second administration, the nasal wash was collected to evaluate the antibody induction in nasal mucosae infected with the influenza virus. Fig. 5A illustrates a virus-specific IgG titer in the nasal cavity against an A/Singapore/GP1908/2015 strain, and the 1-month-old mice showed a significantly higher antibody induction in the inactivated whole-virus vaccine-administered group (W.V.) than in the split vaccine-administered group (S.V.). However, the 18-month-old mice showed comparable antibody titers in any vaccine administration. Fig. 5B illustrates a virus-specific IgG titer in the nasal cavity against a B/Phuket/3073/2013 strain, and it was confirmed that both the 1-month-old mice and the 18-month-old mice showed a significantly higher antibody titer in the inactivated whole-virus vaccine-administered group (W.V.) than in the split vaccine-administered group (S.V.).

From the results described above, in the pediatric and elderly model animals, a significantly higher antibody induction in the serums was confirmed for the inactivated whole-virus vaccine than the split vaccine approved. Furthermore, the antibody induction in the nasal mucosa as a site infected by the virus was significantly higher in the inactivated whole-virus vaccine-administered group (W.V.) than in the split vaccine-administered group (S.V.) against both the type-A virus and the type-B virus evaluated in the present example in the pediatric model animals. Although the antibody induction against the type-A virus was comparable, a significantly higher antibody induction was showed with the inactivated whole-virus vaccine against the type-B virus in the elderly model animals. In other words, a significant improvement in immunogenicity was shown in both the pediatric model animals and the elderly model animals, and it is considered that the beta-propiolactone-treated inactivated whole-virus vaccine shows a higher efficacy than the split vaccine approved.

## Claims

1. An influenza vaccine composition to be administered to children and/or elderly people, comprising, as a virus antigen, inactivated whole particles subjected to inactivation treatment with beta-propiolactone.

2. The composition according to claim 1, wherein the virus antigen contains an influenza A virus strain including an A/H1N1 subtype and an A/H3N2 subtype, and an influenza B virus strain including a B/Victoria lineage and a B/Yamagata lineage.

3. The composition according to claim 1 or 2, comprising no adjuvant.

4. The composition according to any one of claims 1 to 3, wherein the composition is subcutaneously administered.

5. An immunization method comprising administering a composition containing, as a virus antigen, inactivated whole particles subjected to inactivation treatment with beta-propiolactone to children or elderly people.

6. An influenza prevention method comprising administering a composition containing, as a virus antigen, inactivated whole particles subjected to inactivation treatment with beta-propiolactone to children or elderly people.
